# EUROPEAN PATENT APPLICATION

(11) **EP 3 636 659 A1**
(43) Date of publication of application: **15.04.2020**
(21) Application number: 18382707.0
(22) Date of filing: 08.10.2018
(51) Int. Cl.: C07K 7/62, A61K 38/12

(54) **POLYMYXIN-BASED COMPOUNDS USEFUL AS ANTIBACTERIAL AGENTS**

(71) Applicant: Universitat de Barcelona, 08028 Barcelona (ES)
(72) Inventor: RABANAL ANGLADA, Francesc, 08028 Barcelona (ES)

(57) **Abstract**

It relates to polymyxin analogues having a disulfide bond and an amino alcohol group adjacent to the disulfide bond, and optionally, an ester bond in the peptide backbone, which are useful as antibacterial agent, as well as to pharmaceutical compositions comprising them. It also relates to a key intermediate compound of formula (II) and its preparation process.

## Description

The present invention relates to compounds that are active against multi-drug resistant bacteria, to a preparation process thereof, to pharmaceutical compositions containing them, and to their use in the prophylaxis and/or treatment of bacterial infections.

### BACKGROUND ART

Disease-causing microbes that have become resistant to antibiotic drug therapy are an increasing public health problem. Part of the problem is that bacteria and other microbes that cause infections are remarkably able to develop resistance to antimicrobial drugs. Among the world's most dangerous superbugs are multi-drug resistant bacteria, particularly of the so-called ESKAPE group (*Enterococcus faecium, Staphylococcus aureus, Klebsiella pneumoniae, Acinetobacter baumannii, Pseudomonas aeruginosa,* and *Enterobacter species).*

Natural cyclic antimicrobial peptides (AMPs) have become very important drugs in the treatment of bacterial infections. Polymyxin B (PxB) is a lipopeptide antibiotic isolated from *Paenibacillus polymyxa.* Its basic structure consists of a polycationic peptide ring from which is hanging a tripeptide side chain with a fatty acid tail. Polymyxin B has re-emerged in medical practice in recent years and its use will likely continue to increase due to the worldwide increasing prevalence of nosocomial infections caused by multi-drug resistant bacteria. Polymyxin B and other members of the polymyxin family are drugs of last resort to treat multidrug-resistant infections and sometimes are the only available active antibiotics.

Unfortunately, polymyxins show nephrotoxicity and neurotoxicity problems in clinical practice. It is known that PxB tends to accumulate over long periods (at least 48h) in kidneys long after administration and after it has become barely detectable in serum (i.e. more than 30 time the serum concentration after 6h). This suggests a selective uptake process in renal cells. Moreover, accumulation of PxB in kidneys could be correlated to its nephrotoxicity potential. The mechanism of polymyxin-induced nephrotoxicity is not clear. The structure-activity data on polymyxin analogs seemed to indicate that toxicity was related to the amphipathic nature of the molecule, due to the presence of hydrophobic residues and, particularly, due to the presence of charged Dab side chains at physiological pH.

Previous attempts of reducing the toxicity by shortening the hydrophobic chain have resulted in loss of activity. T. Velkov et al., in "Structure- Activity Relationships of Polymyxin Antibiotics" J. Med. Chem., 2010, vol.53, pp.1898-1916, discloses several medicinal chemistry strategies assayed with the aim of improving the activity profile of polymyxins included targeted modifications of the Dab side chains, modification to the cyclic peptide ring and N^{α} fatty acyl chain derivatives. The results suggest that many of the aforementioned modifications generate poorly active compounds. In the case of modifications to the N^{α}fatty acyl chain, this document explains that the N-terminal fatty acyl chain is crucial for the antimicrobial activity of polymyxins and that the toxicity of the polymyxin can partly be attributed to this chain. It also explains that the activities of N-terminal fatty acyl derivatives containing C9-C14 unbranched fatty acyl chains showed that the longer fatty acyl derivatives were more active against polymyxin-resistant strains and Gram positive bacteria whereas the C10 and C12 derivatives were most effective against the polymyxin susceptible strains. On the other hand, polymyxin analogues with a short (<C7) unbranched fatty acyl chain displayed an antimicrobial activity lower than polymyxin B. N. Katsuma et al., in "Development of Des-Fatty Acyl-Polymyxin B Decapeptide Analogs with Pseudomonas aeruginosa-Specific Antimicrobial Activity", Chem. Pharm. Bull., 2009, vol. 57, pp. 332-336, discloses several N-terminal analogs of des-fatty acyl-polymyxin B (Des-FA-[X]-PMB). The results indicated that analogs in which X is Lys, Arg, Leu or Ala did not have increased antimicrobial activity against the *E. coli, S-Typhimurium* and *P-aeruginosa* compared with Des-Fa-[Dab]-PMB and polymyxin B, and Des-FA-[Trp]-PMB and Des-FA-[Phe]-PMB had reduced activity against *P*. *aeruginosa.*

Some recent attempts of reducing the toxicity of the polymyxin focus the research on analogues of polymyxin having a cysteine disulfide bond obtained from cysteines in position 4 and 10 in the peptide backbone and on introducing additional modifications in the lateral chains or in the peptide backbone to obtain at the same time low toxicity and good bacterial activity (cf. WO2010029196A1, WO2011110716A2, WO2014167160A1, and WO2017093210A1). The disulfide bond between the two cysteines and, the optional ester bond in the macrocycle provided an easily metabolizable scaffold. The metabolizable scaffold was expected to yield a lower toxicity since would prevent accumulation in renal cells. The eventual uptake of these analogs by renal cells would presumably reduce the disulfide between cysteines and would open up the peptide cycle due to the reducing intracellular environment (by the presence of reduced glutathione and oxidorreductases) and thus would facilitate the proteolysis of the linear sequence. The derivatives disclosed in WO2010029196A1, WO2011110716A2, WO2014167160A1 maintained a long N^{α}fatty acyl chain (C₉-C₁₂) which was known to produce certain toxicity but which was needed to achieve a good antibacterial activity. The analogs of polymyxin disclosed in WO2017093210A1 had a shorter length fatty acyl unit than polymyxin or simply there was a lack of such unit, but included a hydrophobic alkyl chain at the 1^{st} and 6^{th} amino acid positions.

Some of the analogues disclosed in these documents showed good antibacterial activity. Unfortunately, contrary to the expectation of the most promising analogue (Decanoyl-Dab-Thr-Dab-ciclo(S-S)[Cys-Dab-DPhe-Nle-Dab-Dab-DCys), which did not showed toxicity in acute toxicity tests, it showed toxicity in the nephrotoxicity tests carried out in mice. As it is shown in the comparative examples of the present document, the following compounds disclosed in the previous documents (Nonanoil-Dab-Thr-Dab-cyclo(S-S)[Cys-Dab-DPhe-Leu-Dab-Dab-Cys] (disclosed in Scientific Reports 5:10558 compound 1); Decanoyl-Dab-Thr-Dab-cyclo(S-S)[Cys-Dab-DPhe-Leu-Dab-Dab-DCys] (disclosed in Scientific Reports 5:10558 compound 38); Heptanoyl-Dab-Thr-Dab-cyclo(S-S)[Cys-Dab-DAoc-Nle-Dab-Dab-DCys] (disclosed in WO2017093210A1 Example 1); DAoc-Thr-Dab-cyclo(S-S)[Cys-Dab-DAoc-Leu-Dab-Dab-DCys] (disclosed in WO2017093210A1 Example 9); and (5) DAoc-Thr-Dab-cyclo(S-S)[Cys-Dab-DNle-Leu-Dab-Dab-DCys]) showed nephrotoxicity. From the previous attempts it is derived that the introduction of the disulfide bond between the two cysteines and optionally, the ester bond in the macrocycle of the previous analogs is not enough to obtain compounds without toxicity. Thus, there is a clear challenging unmet medical need to find high effective multi-drug resistant antibacterial agents with low toxicity.

### SUMMARY OF THE INVENTION

Inventors have developed some analogs of polymyxin with reduced nephrotoxicity and with high antibacterial activity against multi-drug resistant bacteria, in particular, gram-negative bacteria. The inventors surprisingly found that nephrotoxicity could be reduced and the antibacterial activity maintained by introducing a disulfide bond, and optionally, an ester bond in the peptide backbone of polymyxin, provided that there is an amino alcohol fragment adjacent to the disulfide bond instead of a cysteine amide group. Unlike the compounds of the present invention, the compounds disclosed in WO2010029196A1, WO2011110716A2, WO2014167160A1, and WO2017093210A1 were prepared from natural amino acids through peptide synthesis and had a C-terminal amide group adjacent to the disulfide bond.

Thus, it has now been found that that there is only the need of incorporating a disulfide bond without additionally modifying the threonine side chain to a C-terminal amide to reduce the toxicity while maintaining the antibacterial activity. This is unexpected since all the previous attempts of providing polymyxin analogues having a disulfide bond in the peptide backbone, failed in showing lack of toxicity. As mentioned above, despite the acute toxicity tests of the most promising polymyxin analogue seemed to give good results, the subsequent nephrotoxicity tests carried out to such compound unexpectedly showed that it was nephrotoxic like it is polymyxin B. Thus, one skilled in the art would have considered that the disulfide bridge in the peptide backbone is not sufficient to obtain good antibacterial activity without nephrotoxicity. Because of this different behavior observed on structurally similar compounds, the finding of some analogues with a different behavior in terms of nephrotoxicity is unexpected since this could not be known until the compound is tested in mice.

Unlike the compounds disclosed in WO2010029196A1, WO2011110716A2, WO2014167160A1 or WO2017093210A1, the compounds of the present invention cannot be obtained from natural amino acids in its entirety. However, the present inventors have now found a method of chemical synthesis to prepare a key building block (compound of formula (II) where PG₁ is an amino protective group and PG₂ is an alcohol protective group) for the introduction of the amino alcohol into the peptide backbone of these compounds.

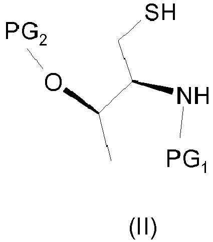

Thus, an aspect of the present invention relates to compounds of formula (I), 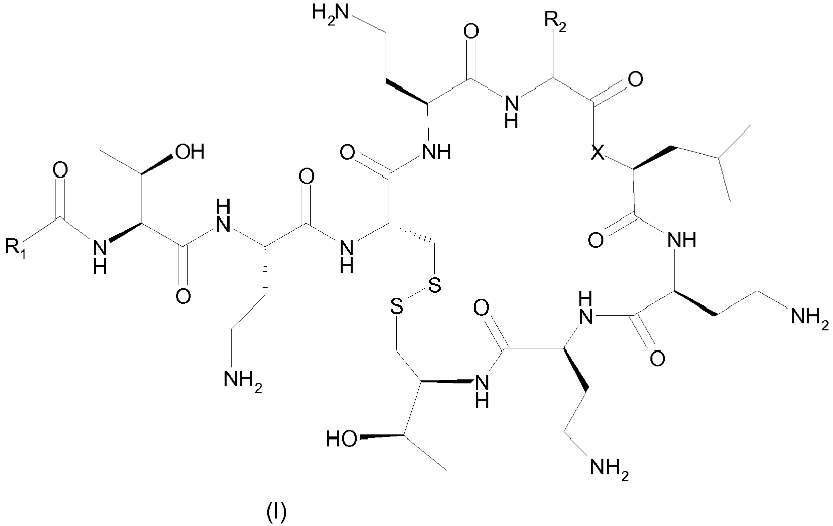 wherein: R₁ is a radical selected from the group consisting of:

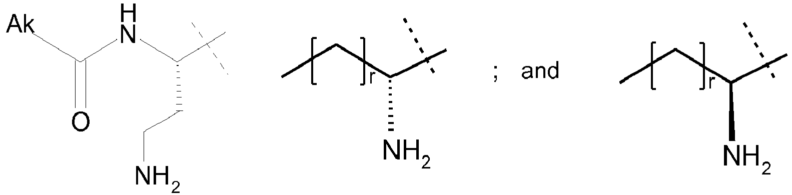

Ak means a (C₆-C₁₁)-alkyl which may be linear or branched; r is an integer from 5 to 9; X is NH or O; and R₂ is selected from the group consisting of a linear or branched (C₃-C₆)-alkyl radical, phenyl, indol, 4-fluorophenyl, and pentafluorophenyl.

Another aspect of the present invention relates to the compounds as defined above, for use as antibacterial agent.

Another aspect of the present invention relates to a pharmaceutical composition comprising a therapeutically effective amount of a compound (I) as defined above, together with appropriate pharmaceutically acceptable excipients or carriers.

Another aspect of the present invention relates to a combination of a therapeutically effective amount of a first antibiotic which is compound (I) as defined above with at least one or more known antibiotics, for use in the prophylaxis and/or treatment of a bacterial infection, wherein the prophylaxis and/or treatment comprises administering the antibiotics to a subject simultaneously, sequentially or separately.

Another aspect of the present invention relates to a pharmaceutical composition comprising a therapeutically effective amount of a compound of formula (I) as defined above and a therapeutically effective amount of one or more known antibiotics, together with pharmaceutically acceptable excipients or carriers, which is a single dosage form; or alternatively, a kit of parts comprising a pharmaceutical composition comprising a therapeutically effective amount of the compound of formula (I) together with pharmaceutically acceptable excipients or carriers, and one or more pharmaceutical compositions comprising a therapeutically effective amount of a known antibiotic together with pharmaceutically acceptable excipients or carriers.

The compounds of formula (I) of the present invention can be easily prepared by chemical synthesis together with solid phase techniques for peptide synthesis in good yields. The process for preparing the compounds of formula (I) as defined above comprises the use of a key intermediate of formula (II) wherein PG₁ and PG₂ are an amino and an alcohol protective group respectively.

Thus, another aspect of the present invention relates to the key intermediate compound of formula (II) wherein PG₁ is an amino protective group and PG₂ is an alcohol protective group. In compound of formula (II), PG₁ and PG₂ are different protective groups, i.e. they have different values.

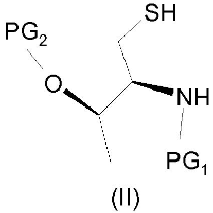

This key intermediate can be prepared by chemical synthesis. Therefore, another aspect of the present invention relates to the process for preparing the compound of formula (II) which comprises submitting a compound of formula (III) wherein PG₁ is an amino protective group and PG₂ is an alcohol protecting group to a deprotection reaction of the thioester group to yield the compound of formula (II) as defined above.

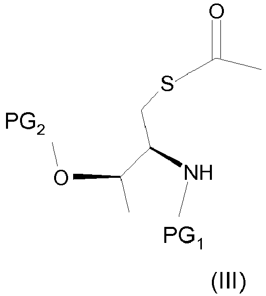

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

As mentioned above, an aspect of the present invention relates to compounds of formula (I) as defined above. In a particular embodiment of this first aspect, the compounds of formula (I) are those where R₁ is a radical selected from the group consisting of:

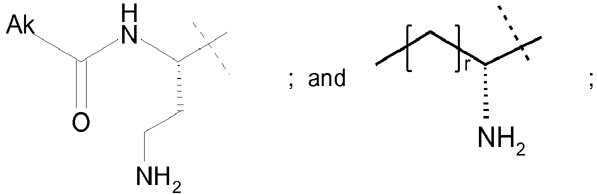

Ak means a (C₆-C₁₁)-alkyl which may be linear or branched; r is an integer from 5 to 9; X is NH or O; and R₂ is a linear or branched (C₃-C₆)-alkyl radical, or phenyl.

In another particular embodiment, compounds of formula (I) are those where Ak is a linear (C₆-C₁₁)-alkyl, thus, in a particular embodiment, compounds of formula (I) are those where: R₁ is a radical selected from the group consisting of: 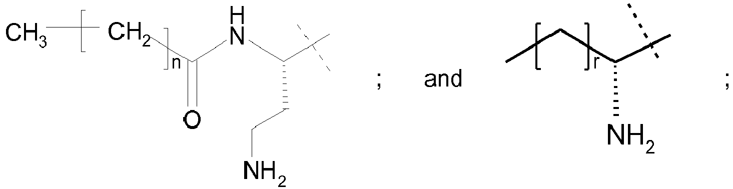 n is an integer from 5 to 10, R₂ is a linear or branched (C₃-C₆)-alkyl radical or phenyl. and r and X are as defined above.

In another particular embodiment, compounds of formula (I) are those where Ak is a linear (C₆-C₉)-alkyl. In another particular embodiment, the compound of formula (I) is that wherein in R₁ n is an integer from 5 to 8.

In another particular embodiment, compounds of formula (I) are those where R₁ is the radical below and n is 6.

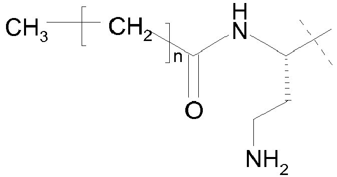

In another particular embodiment, compounds of formula (I) are those where R₁ is the radical below and r is 7.

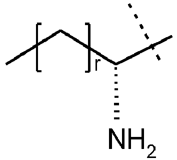

In a particular embodiment, compounds of formula (I) described above are those where R₂ is isobutyl or phenyl.

In another particular embodiment, compound of formula (I) are those where X is NH. In another particular embodiment, compounds of formula (I) are those where X is O.

In another particular embodiment, compounds of formula (I) are those where R₁ and R₂ are as defined above and has the following stereochemistry of the R₂:

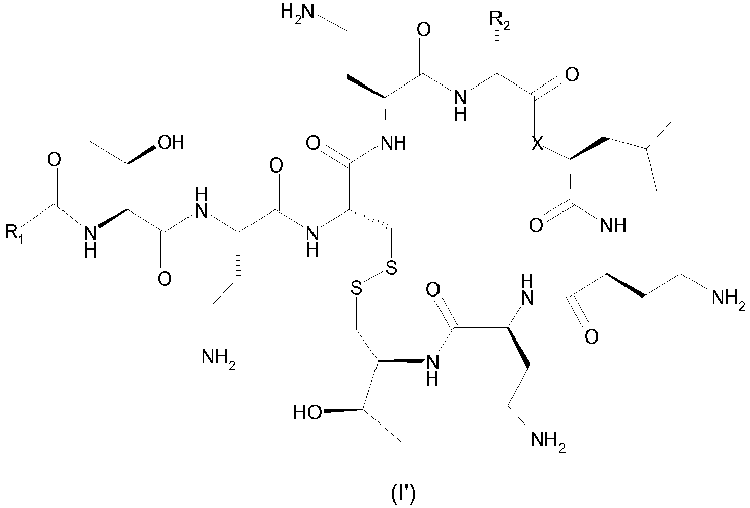

All the particular embodiments described above for compounds of formula (I) are also particular embodiments for the previous compound of formula (I) with the specified stereochemistry of the R₂ substituent.

In another particular embodiment, the compound of formula (I) is that which is selected from the following list:
DAdec-Thr-Dab-cyclo(S-S)[Cys-Dab-DPhe-Leu-Dab-Dab-Ttr], (Ia);
DAdec-Thr-Dab-cyclo(S-S)[Cys-Dab-DLeu-Leu-Dab-Dab-Ttr], (Ib);
Octanoyl-Dab-Thr-Dab-cyclo(S-S)[Cys-Dab-DLeu-OLe-Dab-Dab-Ttr], (Ic);
DAdec-Thr-Dab-cyclo(S-S)[Cys-Dab-DLeu-OLe-Dab-Dab-Ttr], (Id);
Octanoyl-Dab-Thr-Dab-cyclo(S-S)[Cys-Dab-DPhe-OLe-Dab-Dab-Ttr], (Ie);
DAdec-Thr-Dab-cyclo(S-S)[Cys-Dab-DPhe-OLe-Dab-Dab-Ttr]; (If).

The combination of the values of any of the particular embodiments of the compounds of formula (I) are also part of the invention.

The compounds can be easily prepared by chemical synthesis together with solid-peptide synthesis in good yields. In particular, they can be prepared by Fmoc/tBu solid phase peptide synthesis according to the general methods included in the experimental section. The analogues of polymyxin of formula (I) of the present invention can be prepared by solid-phase peptide synthesis as disclosed in the Examples using the intermediate of formula (II). The compounds of formula (I) obtained can be purified by preparative HPLC with which a final purity greater than 99% can be achieved by this method.

The intermediate compound of formula (II) is prepared by chemical synthesis The process for preparing the compound of formula (II) may be carried out starting from threonine protected with an amino protective group (PG₁) and an alcohol protective group (PG₂) as starting material. The process comprises several steps. Basically the process comprises submitting protected threonine (compound of formula (V)) to a reduction reaction of the carboxyl group to a hydroxyl group, to yield a compound of formula (IV), wherein in compound (V) and (IV), PG₁ is an amino protective group and PG₂ is an alcohol protective group.

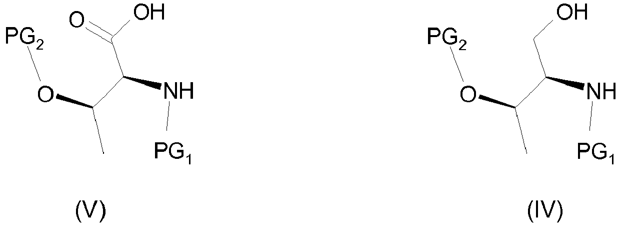

Then, submitting the intermediate (IV) thus obtained to a Mitsunobu reaction in the presence of thioacetic acid to yield a compound of formula (III), wherein PG₁ is an amino protective group and PG₂ is an alcohol protective group.

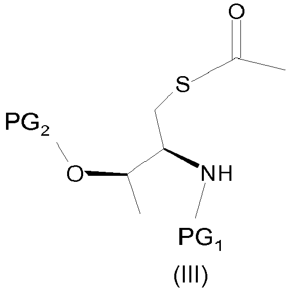

Finally, the following step is submitting the compound of formula (III) to a deprotection reaction of the thioester group to yield the compound of formula (II), wherein PG₁ is an amino protective group and PG₂ is an alcohol protective group. The deprotection reaction may be an aminolysis or other suitable deacetylation reaction.

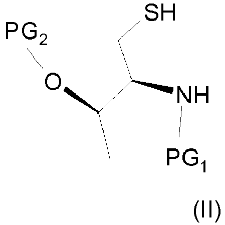

In compounds (II), (III), (IV) and (V), PG₁ and PG₂ are compatible protective groups, which means that they have different values and can be introduced and removed in different conditions. In a particular embodiment, the amino protective group of the starting material (V) as well as of the intermediates (IV), (III) and (II) is selected from carbamates, amides, sulfonamides, of appropiate allyl, benzyl and a mono or a di-substituted benzyl groups. In another particular embodiment, the amino protective group is selected from 9-fluorenylmethoxycarbonyl (Fmoc), allyl, 4-methoxybenzyl, 2,4-dimethoxybenzyl and N-benzyl carbamate. In a preferred embodiment, the PG₁ is 9-fluorenylmethoxycarbonyl (Fmoc).

The specific conditions depend on the protective group used. In a particular embodiment, when an Fmoc group is used, it can be introduced by reaction of Fmoc-CI or Fmoc-osuccinimidyl in the presence of a suitable solvent and an organic or inorganic base. Deprotection takes place under mild conditions by reaction with a base. Suitable bases for the deprotection are any organic or inorganic base, for example, piperidine, morpholine, dicyclohexylamine, K₂CO₃ or KHCO_{3.}. In a particular embodiment the protective group is Fmoc and the deprotection reaction is for instance carried out using piperidine in N,N-dimethylformamide.

The PG₂ is in compounds (II), (III), (IV) and (V) an alcohol protective group. In a particular embodiment, the alcohol protective group of the starting material (V) as well as of the intermediates (IV), (III) and (II) is selected from tert-butyl, allyl, and benzyl. In a preferred embodiment, the PG₂ is tert-butyl. The specific conditions depend on the protective group used. In a particular embodiment, when a tertbutyl group is used, it can be introduced for instance by reaction of isobutylene, BF₃. Et₂O, H₃PO₄. The cleavage can for instance be carried out with CF₃COOH or HBr/AcOH.

The introduction and removal of the protecting groups PG₁ and PG₂ can be carried out by methods known in the art (cf. Protective Groups in Organic Synthesis, Wiley-Interscience, 1999 chapter 7 or A.I. Llobet et al., "Amino Acid- Protecting Groups", Chem Rev 2009, vol. 109, pp. 2455-2504).

PG₁ and PG₂ must be compatible protective groups which means that can be deprotected in different conditions. For instance, PG₂ can be tert-butyl and PG₁ can be Fmoc, or PG₂ can be Bzl and PG₁ can be Boc.

The reduction of the carboxyl group to a hydroxy group of the threonine protected with a protective group (compound of formula (V)) to yield compound of formula (IV) may be carried out by its reaction with carbonyldiimidazole in an appropriate solvent such as a polar solvent, followed by reaction with a reducing agent such as NaBH₄. In another particular embodiment, the solvent of the reaction with carbonylimidazole is selected from acetonitrile, methanol, ethanol, and tetrahydrofuran, In another particular embodiment, the solvent of the reaction with carbonylimidazole is tetrahydrofuran. Generally, this reaction is carried out at room temperature (T= 20-25 °C). Generally, the reaction with NaBH₄ is carried out in tetrahydofuran or a mixture of tetrahydrofuran/water. Generally, this reduction reaction is carried out at a temperature comprised between 0-5 °C.

The Mitsunobu reaction may be carried out by reaction of a compound of formula (IV) with azodicarboxylate, triphenylphosphine, and thioacetic acid in the presence of an appropriate solvent to yield the compound of formula (III). The solvent is preferably a polar solvent. In a particular embodiment, the solvent is tetrahydrofuran. Generally, the reaction is carried out at a temperature comprised between 0-5 °C. In a particular embodiment, the azodicarboxylate is diethyl azodicarboxylate or diisopropyl azodicarboxylate.

The compound (III) thus obtained is converted in compound of formula (II) by reaction with NH₂OH and tris(2-carboxyethyl)phosphine (TCEP) in an appropriate solvent. In a particular embodiment, the appropriate solvent is CH₃CN.

In a particular embodiment, the compound of formula (V) is the compound (Va).

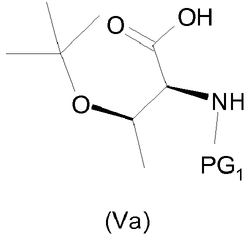

In another particular embodiment, the compound of formula (IV) is the compound of formula (IVa).

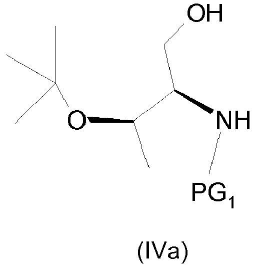

In another particular embodiment, the compound of formula (III) is the compound of formula (IIIa).

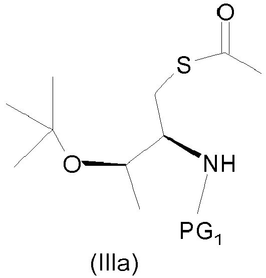

In another particlar embodiment the compound of formula (II) is the compound of formula (IIa).

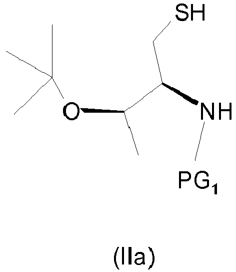

In compounds (Va), (IVa), (IIIa), and (IIa), PG₁ is an amino protective group. In a preferred embodiment, compounds of formula (V), (IV), (III), and (II) are those where PG₁ is 9-fluorenylmethoxycarbonyl and PG₂ is tert-butyl.

As mentioned above, it is part of the invention the compounds of formula (I) as defined above for use as antibacterial agents. In a particular embodiment, the compounds are for use as antibacterial agents against resistant bacteria. In another particular embodiment, the compounds are for use as antibacterial agents against multi-drug resistant bacteria. In another particular embodiment, the compounds are for use as antibacterial agents against gram-negative bacteria. Medically relevant for the purposes of the invention are *Escherichia coli, Klebsiella pneumoniae,* and *Pseudomonas aeruginosa.* This aspect of the invention can also be formulated as use of a compound of formula (I) as defined above for the preparation of a medicament for the prophylaxis and/or treatment of a bacterial infection, in particular caused by resistant bacteria, or by gram-negative bacteria, more in particular caused by caused by multi-drug resistant bacteria, in a mammal, including a human. The invention also relates to a method of treatment and/or prophylaxis of a bacterial infection in a mammal, including a human, suffering from or being susceptible to the bacterial infection, in particular caused by resistant bacteria, or by gram-negative bacteria, more in particular caused by multi-drug resistant bacteria, the method comprising the administration to the patient of a therapeutically effective amount of the compounds of formula (I) as defined above, together with pharmaceutically acceptable excipients or carriers.

Polymyxin derivatives can disrupt the bacterial outer membrane (OM) and facilitate the access of hydrophobic antibiotics and large hydrophilic antibiotics, such as vancomyin, rifampicin, erythromycin, clarithromycin, tetracyclines, and carbapenems such as imipenem, doripenem and meropenem. Compounds of the present invention can be used analogously to other known polymyxin derivatives. They may be used alone or in combination with other suitable bioactive compounds. In a particular embodiment, compounds of formula (I) can be used in the prophylaxis and/or treatment of internal and topical infections, following infection by bacteria for instance, bacteremia and/or septicemia, pneumonia, urinary and intra-abdominal tract infections (UTI, IAI), endocarditis, SSSI (skin and skin structure infections), etc, administered alone or in combination with conventional antibiotics. Thus, it is part of the invention a combination of a therapeutically effective amount of a first antibiotic which is a compound of formula (I) as defined above and a therapeutically effective amount of one or more known antibiotics, for use in the prophylaxis and/or treatment of a bacterial infection, wherein the prophylaxis and/or treatment comprises administering the antibiotics to a subject simultaneously, sequentially or separately.

It is also part of the invention a compound of formula (I) as defined above for use in combination therapy for the prophylaxis and/or treatment of a bacterial infection, wherein said medicament is to be administered in combination with one or more known antibiotics. The known antibiotics can be a hydrophobic antibiotic or a large hydrophilic antibiotic. Examples of suitable antibiotics for the purposes of the invention are vancomyin, rifampicin, erythromycin, clarithromycin, tetracyclines, penicillin, cephalosporin, and carbapenems such as imipenem, doripenem and meropenem.

In a particular embodiment, the bacterial infection of the previous embodiments is caused by resistant bacteria. In another particular embodiment, the bacterial infection of the previous embodiments is caused by multi-drug resistant bacteria. In another particular embodiment, the bacterial infection of the previous embodiments is caused by gram-negative bacteria. Medically relevant for the purposes of the invention are *Escherichia coli, Klebsiella pneumoniae,* and *Pseudomonas aeruginosa.*

In a particular embodiment, the compounds of formula (I) of the present invention are for topical or oral use for the decontamination of the digestive tract before surgery.

A pharmaceutical composition comprising a therapeutically effective amount of the compounds of formula (I), together with appropriate amounts of pharmaceutically acceptable excipients or carriers is also part of the invention.

The pharmaceutical compositions may be prepared by combining the compounds of formula (I) of this invention with solid or liquid pharmaceutically acceptable excipients or carriers, in accordance with standard pharmaceutical practices.

The pharmaceutical compositions of this invention may be administered in a manner adequate for the disease that it is desired to treat, for example by oral, parenteral, inhalatory, rectal, transdermal or topical administration. In therapeutic use for treating, or combating bacterial infections in patients such as humans and other animals that can be diagnosed with bacterial infections, the compounds or pharmaceutical compositions thereof, preferably, are administered at a dosage to obtain and maintain a concentration, that is, an amount or blood-level of active component in the patient undergoing treatment which will be antibacterially effective. Generally such antibacterially effective amount of dosage of active component will be in the range of about 0.1 to about 100 mg/Kg, more preferably about 3.0 to about 50 mg/Kg of body weight/day. It is to be understood that the dosages may vary depending upon the requirements of the patient, the severity of the bacterial infection being treated, and the particular compound being used.

Pharmaceutical compositions comprising a therapeutically effective amount of a compound of formula (I) as defined above, and a therapeutically effective amount of one or more known antibiotics, together with pharmaceutically acceptable excipients or carriers, are also part of the invention. In a particular embodiment, the pharmaceutical composition of the invention comprises a compound of formula (I) and a second antibiotic as active agents. In another particular embodiment, the pharmaceutical composition comprises a compound of formula (I) and two additional antibiotics as active agents.

The active ingredients can be formulated in a single dosage form or independently. When the active ingredients are formulated independently, the respective formulations can form part of a kit of parts. Thus, a kit comprising a pharmaceutical composition comprising a therapeutically effective amount of a compound of formula (I) together with pharmaceutically acceptable excipients or carriers, and one or more pharmaceutical compositions comprising a therapeutically effective amount of a known antibiotic together with pharmaceutically acceptable excipients or carriers, is also part of the invention.

Finally, the present invention covers all the possible combinations of particular and/or preferred embodiments previously mentioned. Throughout the description and claims the word "comprise" and variations of the word are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention.

### EXAMPLES

Abbreviations: Ac, acetyl, Adec, 2-aminodecanoic acid; Boc, tert-butoxycarbonyl; Dab: 2,3-diaminobutyric acid; CDI, carbonyldiimidazole; CTC, 2-Chlorotrityl chloride; DIEA, N,N-diisopropylethylamine; DIPCDI, N,N'-diisopropylcarbodiimide; DMF, N,N-dimethylformamide; DMSO, dimethylsulfoxide; ESI: electrospray ionization; Fmoc, 9-fluorenylmethoxycarbonyl; HOBt, 1-hydroxybenzotriazole; HPLC, high performance liquid chromatography; MALDI-TOF, matrix-assisted laser desorption ionization time-of-flight; MS, mass spectrometry; MIC, minimum inhibitory concentration; MW, molecular weight; OLe, leucic acid residue; sc. subcutaneous (administration); tBu, tert-butyl; TCEP, tris(2-carboxyethyl)phosphine; TFA, trifluoroacetic acid; THF, tetrahydrofuran; Trt, trityl; Ttr, (2*R*,3*S*)-3-amino-4-mercaptobutan-2-ol (Thiothreonine)

### Example 1: Preparation of Fmoc-L-Thr(tBu)-CH₂OH

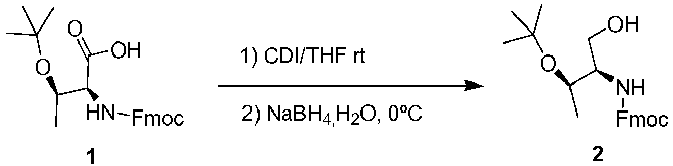

Fmoc-L-Thr(tBu)-CH₂OH (compound 2) was prepared according to well-established procedures. Carbonyldiimidazole, CDI (9.2 g, 56.6 mmol), was added to a stirred solution of Fmoc-L-Thr(tBu)-OH 1 (15.0 g, 37.7 mmol) in THF (90 mL) at room temperature. The reaction was led to completion by addition of small amounts of CDI according to HPLC-MS monitoring. After 1h the reaction was completed. The solution was cooled to 0°C and a solution of NaBH₄ (2.4 g, 63.0 mmol) in H₂O (60 mL) was added in one portion. The solution was then stirred for an additional hour. Then, 1N aqueous HCI (300mL) was added and the resulting solution was extracted with ethyl acetate (2x300 mL). The combined organic layers were washed with saturated aqueous NaHCO₃ (300 mL) and brine (300 mL). The organic layer was dried over anhidrous MgSO₄, filtered and concentrated *in vacuo.* The crude was purified by flash chromatography over silica with hexane-ethyl acetate to provide 13.1 g of the desired product Fmoc-L-Thr(tBu)-CH₂OH **2** (90.3% yield). MS (ES+): m/z=384.1 [M+H]⁺.

### Example 2: Preparation of Fmoc-L-Thr(tBu)-CH₂-S-COCH₃-

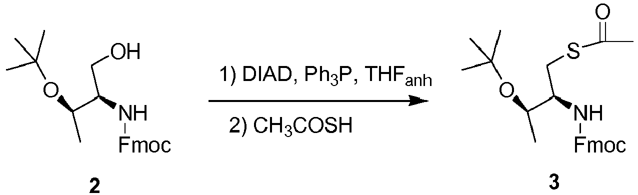

Diisopropyl azodicarboxylate (10.1 mL, 51.1 mmol) was added to a well stirred solution of triphenylphosphine (13.4 g, 51.1 mmol) in 145 ml of tetrahydrofuran anhydrous over activated molecular sieves under argon atmosphere at 0°C. The mixture was stirred at 0°C for 50 minutes. A light yellow precipitate resulted. A mixture of Fmoc-L-Thr(tBu)-CH₂OH **2** (13.1 g, 34.1 mmol) and thioacetic acid (6.1 mL, 85.2 mmol) in 29 ml tetrahydrofuran anhydrous was added dropwise during 1h. The mixture was stirred for 1.5 h at room temperature. The molecular sieves were filtrated and the mixture was concentrated *in vacuo* and purified by flash chromatography over silica with hexane-ethyl acetate to provide 12.7 g of desired thioacetate Fmoc-L-Thr(tBu)-CH₂SCOCH₃ **3** (84.6% yield). MS (ES+): m/z 442.0 [M+H]⁺. ¹H NMR (400 MHz, Chloroform-d) δ 7.78 (m, 2H), 7.6 (m, 2H), 7.4 (m, 2H), 7.32 (m, 2H), 5.08 (d, 1H), 4.38 (m, 2H), 4.22 (t, 1H), 3.82 (m, 1H), 3.68 (m, 1H), 3.05 (m, 2H), 2.37 (s, 3H), 1.22 (s, 9H), 1.07 (d, 3H).

### Example 3: Preparation of Fmoc-L-Thr(tBu)-CH₂-SH (Fmoc-Ttr(^{t}Bu)-CH₂SH)

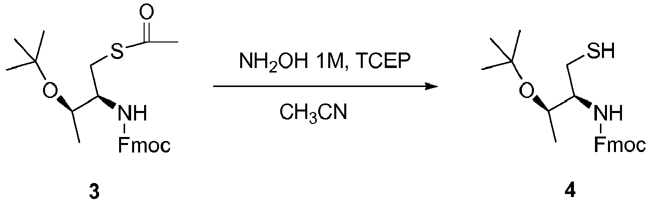

A solution of thioacetate Fmoc-L-Thr(tBu)-CH₂SAc **3** (9 g, 20.4 mmol) in 305 mL of CH₃CN was treated with a solution of 1M NH₂OH (305 mL, 305.7 mmol) and TCEP (8.35 g, 29.1 mmol) yielding a pH= 6.53. The reaction was stirred at room temperature overnight. The CH₃CN was removed under reduced pressure. The solution was extracted with 2 x CH₂Cl₂. The combined organic layers were dried (MgSO4) and concentrated *in vacuo.* The crude was purified by flash chromatography over C18 column with H₂O-CH₃CN (0.1% HCOOH) to provide 7.4 g of the desired product Fmoc-L-Thr(tBu)-CH₂SH 4 (90.9% yield). MS (ES+): m/z 400.0 [M+H]⁺. ¹H NMR (400 MHz, Chloroform-d) δ 7.71 - 7.69 (m, 2H), 7.55 (d, *J* = 7.5 Hz, 2H), 7.35 - 7.31 (m, 2H), 7.27 - 7.23 (m, 2H), 5.08 (d, *J* = 9.2 Hz, 1H), 4.40 - 4.32 (m, 2H), 4.18 (t, , *J* = 7.0 Hz, 1H), 3.96 - 3.90 (m, 1H), 3.43 (q, *J* = 7.7 Hz, 1H), 2.64 - 2.46 (m, 2H), 1.32 (dd, *J* = 9.6, 7.5 Hz, 1H), 1.13 (s, 9H), 1.09 (d, *J* = 6.2 Hz, 3H).

### Example 4: General method for the synthesis of peptides

To prepare the compounds of the Examples, general protocols of Fmoc/tBu solid phase peptide synthesis were used. Manual peptide syntheses were performed using polypropylene syringes fitted with a polyethylene disc. The 2-Chlorotrityl chloride (CTC) resin was used as the solid support.

The Fmoc general synthesis protocol for each amino acid consisted of the following steps: (i) resin washing with DMF (5 x 30 s); (ii) treatment with 20 % piperidine/DMF (1 x 1 min, 2 x 10 min, Fmoc removal); (iii) washing with DMF (5 x 30 s); (iv) acylation with Fmoc-protected amino acid (3 times of excess) with activating reagent DIPCDI/HOBt (3 times of excess for both) in the minimum amount of DMF; (v) washing with DMF (5 x 30 s) and CH₂Cl₂ (5 x 30 s); (vi) Kaiser's test (of a peptide-resin sample); (vii) DMF washing (5 x 30 s). When a positive result was obtained with the Kaiser's test, the Fmoc-protected amino acid (or fatty acid or hydroxy acid) was recoupled with half of the equivalents, i. e. steps (iii) to (vii) were repeated. When leucic acid (a hydroxy acid) was coupled instead of a protected amino acid, step (iv) of the protocol was modified as follows: (iv) leucic acid (2 times of excess) was activated with DIPCDI/HOBt (2 and 4 times of excess, respectively) in the minimum amount of DMF. The reaction was repeated twice. The ester bond was obtained in the following cycle by esterification with the corresponding Fmoc-protected amino acid: iv) esterification with the Fmoc-protected amino acid (6 times of excess) using activating reagent DIPCDI (3 times of excess, 30 min preactivation) and then adding DMAP (0.3 equivalents) in the minimum amount of DMF. The procedure was repeated using additional Fmoc-protected amino acid, DIPCDI and DMAP in proportions (3:1.5:0.15). Completion of the ester bond cannot be monitored with the Kaiser's test in this case, so a pilot cleavage test was carried out with a small peptide-resin sample and monitored by HPLC and ESI-MS. Coupling of the octanoic acid was performed when necessary, using 5 equivalents of excess together with DIPCDI/HOBt (5 times of excess for both) in the minimum amount of DMF. General cleavage and full deprotection of the peptides was carried out by treatment with trifluoroacetic acid/triisopropylsilane/H₂O (95:3:2, v/v, minimum 30 min, repeated when necessary). The TFA solution was filtered off and collected. The resin was rinsed twice with an additional TFA mixture, and the filtrates were combined. The solution was concentrated by means of a stream of nitrogen gas. The oily residue was treated with an excess of dry diethylether to obtain the peptide precipitate. Peptide crudes were washed additionally with ether (twice). The solid peptide was isolated by centrifugation. Peptide crudes were then dissolved in a 3% DMSO solution in water to a concentration of 1 mM or slightly lower for intramolecular disulphide bond formation. Cyclization reaction was carried out with continuous stirring for 24-36 h. Completion of the cyclization was monitored by HPLC and MS. Peptides were purified routinely by preparative HPLC using a Phenomenex® C18 column (25 x 1 cm, 5 µm diameter) eluting with with 0.1% TFA/H₂O and 0.1% TFA/acetonitrile and UV detection at 220 nm (unless otherwise stated). Mass spectra were obtained in positive mode using an ESI instrument *Spectrometer ZQ-Micromass* (Waters).

### Example 5: Preparation of DAdec-Thr-Dab-cyclo(S-S)[Cys-Dab-DPhe-Leu-Dab-Dab-Ttr], la

Protected amino acids: Fmoc-Ttr(^{t}Bu)-CH₂SH, Fmoc-Dab(Boc)-OH, Fmoc-DPhe-OH Fmoc-Leu-OH, Fmoc-Cys(Trt)-OH, Fmoc-Thr(^{t}Bu)-OH, Boc-DAdec-OH. A manual peptide synthesis was performed following standard Fmoc/^{t}Bu procedures described above. The CTC resin (126 mg, 0.18 mmole, f= 1.56 mmole/g of resin) was used. The Fmoc-Ttr(tBu)-CH₂SH was incorporated (157 mg, 0.39 mmol, 2 eq) to the resin, with DIEA (140 microL, 0.82 mmole, 4 eq). After the first coupling, the loading was measured by quantifying the amount of Fmoc removed via spectrophotometry (f=0.8 mmole/g of resin). The weight of crude peptide after cleavage was 73 mg (yield 42%). Purification by HPLC yielded 28.9 mg of pure peptide (yield 17%). Characterization of the purified peptide: Homogeneity (by area integration of HPLC trace) >99%; ESI-MS: m/z 1153.8 ([M+H]⁺, 4%), 577.7 ([M+2H/2]²⁺, 100%), 385.4 ([M+3H/3]³⁺, 73%). MW 1152.65.

### Example 6: Preparation of DAdec-Thr-Dab-cyclo(S-S)[Cys-Dab-DLeu-Leu-Dab-Dab-Ttr], lb

Protected amino acids: Fmoc-Ttr(^{t}Bu)-CH₂SH, Fmoc-Dab(Boc)-OH, Fmoc-Leu-OH, Fmoc-DLeu-OH, Fmoc-Cys(Trt)-OH, Fmoc-Thr(^{t}Bu)-OH, Boc-DAdec-OH. A manual peptide synthesis was performed following standard Fmoc/^{t}Bu procedures. The CTC resin (131 mg, 0.21 mmole, f= 1.56 mmole/g of resin) was used. The Fmoc-Ttr(tBu)-CH₂SH was incorporated (164 mg, 0.41 mmol, 2 eq) to the resin, with DIEA (145 microL, 0.83 mmole, 4 eq) and left to react for 12 hours or overnight. After the first coupling, the loading was monitored by quantifying the amount of Fmoc removed via spectrophotometry (f=0.8 mmole/g of resin). The weight of crude peptide was 110 mg (yield 62%). Purification by HPLC yielded 31 mg of pure peptide (yield 18%). Characterization of the purified peptide: Homogeneity (by area integration of HPLC trace) >99%; ESI-MS: m/z 1120.1 ([M+H]⁺, 1%), 560.7 ([M+2H/2]²⁺, 100%), 374.3 ([M+3H/3]³⁺, 42%). MW 1118.67.

### Example 7: Preparation of Octanoyl-Dab-Thr-Dab-cyclo(S-S)rCys-Dab-DLeu-OLe-Dab-Dab-Ttrl, Ic

Protected amino acids: Fmoc-Ttr(^{t}Bu)-CH₂SH, Fmoc-Dab(Boc)-OH, leucic acid, Fmoc-DLeu-OH, Fmoc-Cys(Trt)-OH, Fmoc-Thr(^{t}Bu)-OH,octanoic acid. A manual peptide synthesis was performed following standard Fmoc/^{t}Buprocedures. The CTC resin (166 mg, 0.26 mmole, f= 1.56 mmole/g of resin) was used. The Fmoc-Ttr(tBu)-CH₂SH was incorporated (207 mg, 0.52 mmol, 2 eq) to the resin, with DIEA (185 microL, 1.09 mmole, 4 eq) and left to react for 12 hours or overnight. After the first coupling, the loading was monitored by quantifying the amount of Fmoc removed via spectrophotometry (f=0.8 mmole/g of resin). The weight of crude peptide after cleavage was 56 mg (yield 24%). Purification by HPLC yielded 27 mg of pure peptide (yield 12%). Characterization of the purified peptide: Homogeneity (by area integration of HPLC trace) >99%; ESI-MS: m/z 1178.2 ([M+H]⁺, 5%), 589.8 ([M+2H/2]²⁺, 100%), 393.6 ([M+3H/3]³⁺, 44%). MW 1176.67.

### Example 8: Preparation of DAdec-Thr-Dab-cyclo(S-S)[Cys-Dab-DLeu-OLe-Dab-Dab-Ttr], ld

Protected amino acids: Fmoc-Ttr(^{t}Bu)-CH₂SH, Fmoc-Dab(Boc)-OH, leucic acid, Fmoc-D-Leu-OH, Fmoc-Cys(Trt)-OH, Fmoc-Thr(^{t}Bu)-OH, Boc-DAdec-OH.

A manual peptide synthesis was performed following standard Fmoc/^{t}Buprocedures. The CTC resin (166 mg, 0.26 mmole, f= 1.56 mmole/g of resin) was used. Fmoc-Ttr(tBu)-CH₂SH was incorporated (207 mg, 0.52 mmol, 2 eq) to the resin, with DIEA (185 microL, 1.09 mmole, 4 eq) and left to react for 12 hours or overnight. After the first coupling, the loading was monitored by quantifying the amount of Fmoc removed via spectrophotometry (f=0.7 mmole/g of resin). The amino acids of the sequence were added following a Fmoc/tBu solid phase synthesis protocol as described above. The weight of crude peptide after cleavage was 60 mg (yield 27%). Purification by HPLC yielded 30 mg of pure peptide (yield 13%). Characterization of the purified peptide: Homogeneity (by area integration of HPLC trace) >99%; ESI-MS: m/z 1120.42 ([M+H]⁺, 17%), 560.9 ([M+2H/2]²⁺, 100%), 374.3 ([M+3H/3]³⁺, 18%). MW 1119.65.

### Example 9: Preparation of Octanoyl-Dab-Thr-Dab-cyclo(S-S)[Cys-Dab-DPhe-OLe-Dab-Dab-Ttrl, le

Protected amino acids: Fmoc-Ttr(^{t}Bu)-CH₂SH, Fmoc-Dab(Boc)-OH, Fmoc-DPhe-OH leucic acid, Fmoc-Cys(Trt)-OH, Fmoc-Thr(^{t}Bu)-OH, octanoic acid. A manual peptide synthesis was performed following standard Fmoc/^{t}Bu procedures. The CTC resin (165 mg, 0.26 mmole, f= 1.56 mmole/g of resin) was used. Fmoc-Ttr(tBu)-CH₂SH was incorporated (206 mg, 0.52 mmol, 2 eq) to the resin, with DIEA (184 microL, 1.08 mmole, 4 eq) and left to react for 12 hours or overnight. After the first coupling, the loading was monitored by quantifying the amount of Fmoc removed via spectrophotometry (f=0.9 mmole/g of resin). The amino acids of the sequence were added following a Fmoc/tBu solid phase synthesis protocol. The weight of crude peptide after was 89 mg (yield 34%). Purification by HPLC yielded 26.2 mg of pure peptide (yield 10%). Characterization of the purified peptide: Homogeneity (by area integration of HPLC trace) >99%; ESI-MS: m/z 1211.3 ([M+H]⁺, 2%), 606.4 ([M+2H/2]²⁺, 44%), 404.6 ([M+3H/3]³⁺, 100%). MW 1210.6.

### Example 10: Preparation of DAdec-Thr-Dab-cyclo(S-S)[Cys-Dab-DPhe-OLeDab-Dab-Ttr], lf

Protected amino acids: Fmoc-Ttr(^{t}Bu)-CH₂SH, Fmoc-Dab(Boc)-OH, leucic acid, Fmoc-Cys(Trt)-OH, Fmoc-Thr(^{t}Bu)-OH, Boc-DAdec-OH. A manual peptide synthesis was performed following standard Fmoc/^{t}Bu procedures. The CTC resin (165 mg, 0.26 mmole, f= 1.56 mmole/g of resin) was used. Fmoc-Ttr(tBu)-CH₂SH was incorporated (206 mg, 0.52 mmol, 2 eq) to the resin, with DIEA (184 microL, 1.08 mmole, 4 eq) and left to react for 12 hours or overnight. After the first coupling, the loading was monitored by quantifying the amount of Fmoc removed via spectrophotometry (f=0.9 mmole/g of resin). The amino acids of the sequence were added following a Fmoc/tBu solid phase synthesis protocol. The weight of crude peptide after cleavage was 103 mg (yield 40%). Purification by HPLC yielded 29.5 mg of pure peptide (yield 12%). Characterization of the purified peptide: Homogeneity (by area integration of HPLC trace) >99%; ESI-MS: m/z 1154.4 ([M+H]⁺, 2%), 577.8 ([M+2H/2]²⁺, 71%), 385.6 ([M+3H/3]³⁺, 100%). MW 1153.64.

**Table 1: Summary of the compounds of the invention of the previous examples**

| Example | Compound |
|---|---|
| Example 5 | DAdec-Thr-Dab-*cyclo*(*S*-*S*)[Cys-Dab-DPhe-Leu-Dab-Dab-Ttr] (Ia) |
| Example 6 | DAdec-Thr-Dab-*cyclo*(*S-S*)[Cys-Dab-DLeu-Leu-Dab-Dab-Ttr] (Ib) |
| Example 7 | Octanoyl-Dab-Thr-Dab-*cyclo*(*S*-*S*)[Cys-Dab-DLeu-OLe-Dab-Dab-Ttr] (Ic) |
| Example 8 | DAdec-Thr-Dab-*cyclo*(*S*-*S*)[Cys-Dab-DLeu-OLe-Dab-Dab-Ttr] (Id) |
| Example 9 | Octanoyl-Dab-Thr-Dab-*cyclo*(*S-S*)[Cys-Dab-DPhe-OLe-Dab-Dab-Ttr] (Ie) |
| Example10 | DAdec-Thr-Dab-*cyclo*(*S-S*)[Cys-Dab-DPhe-Ole-Dab-Dab-Ttr] (If) |

**Table 2: Summary of the comparative Examples**

| Comparative Examples | Compound | |
|---|---|---|
| Comparative Example 1 | Nonanoyl-Dab-Thr-Dab-*cyclo*(S-S)[Cys-Dab-DPhe-Leu-Dab-Dab-Cys] | disclosed in Scientific Reports 5:10558 as compound 1 |
| Comparative Example 2 | Decanoyl-Dab-Thr-Dab-*cyclo*(S-S)[Cys-Dab-DPhe-Nle-Dab-Dab-DCys] | disclosed in Scientific Reports 5:10558 as compound 38 |
| Comparative Example 3 | Heptanoyl-Dab-Thr-Dab-*cyclo*(S-S)[Cys-Dab-DAoc-Nle-Dab-Dab-DCys] | disclosed in WO2017093210A1 Example 1 |
| Comparative Example 4 | DAoc-Thr-Dab-*cyclo*(S-S)[Cys-Dab-DAoc-Leu-Dab-Dab-DCys] | disclosed in WO2017093210A1 Example 9 |
| Comparative Example 5 | DAoc-Thr-Dab-*cyclo*(S-S)[Cys-Dab-DNle-Leu-Dab-Dab-DCys] | - |
| Col (PxE). | Colistin | |
| PxB | Polymyxin B | |

### Example 11: Antibacterial test

The antibacterial activity of the polymyxin lipopeptides was measured at ISGlobal following the microdilution test to determine the Minimal Inhibitory Concentrations (MIC) in sterile 96-well polypropilene plates. The concentration range was 32 a 0,06 g/ml in Iso-Sensitest broth (Oxoid, ThermoFisher). The results were carried out in triplicate and the highest MIC is the one shown. Bacterial strains were grown in blood agar plates at 37 °C for 17 h. Fresh colonies were used to prepare a McFarland 0,5 solution that was finally added to the plates at 1 to 100 dilution

Samples of sensitive and multi-drug resistant bacteria were: *E. coli* ATCC 25922 *E. coli* MB1410; *K. pneumoniae* MB674; *P. aeruginosa* ATCC 27853; *P. aeruginosa* 36A.

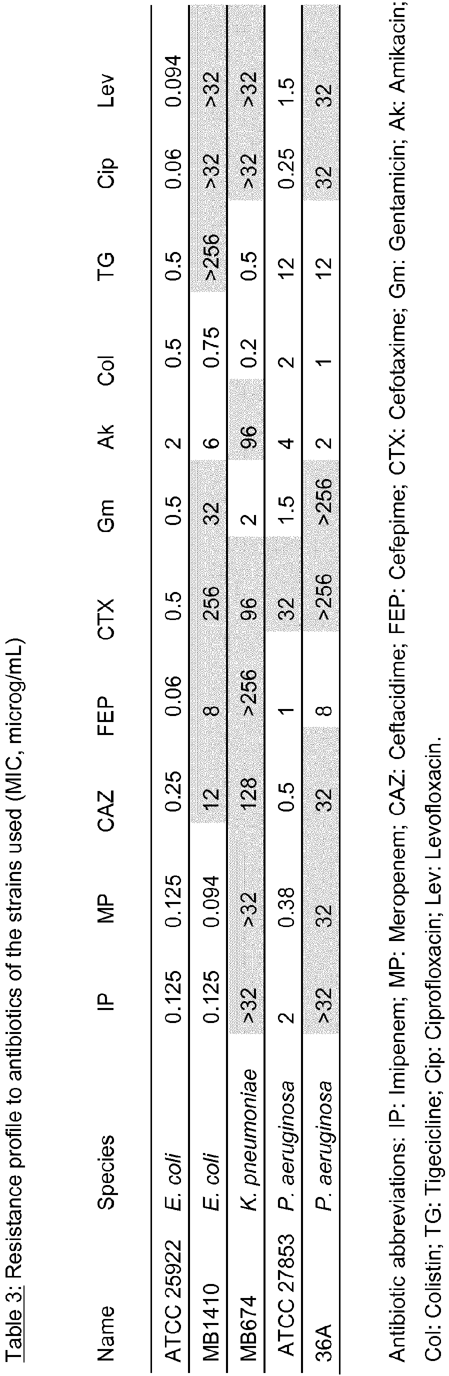

Highlighted cells mean that the strain is resistant to the corresponding antibiotic.

The results obtained in the antibacterial test for examples 5-10 are shown in Tables 4-6.

**Table 4: antibacterial activity (MIC) expressed in µg/ml of the Examples of the invention**

| Strains | Ex. 5 | Ex. 6 | Ex. 7 | Ex.8 | Ex.9 | Ex.10 |
|---|---|---|---|---|---|---|
| *E coli* ATCC 25922 | 1 | 2 | 8 | 2 | 4 | 2 |
| *E.coli* MB1410 | 1 | 0.5 | 4 | 1 | 1 | 1 |
| *K. pneumoniae* MB674 | 1 | 0.5 | 1 | 1 | 1 | 2 |
| *P. aeruginosa* ATCC 27853 | 2 | 1 | 2 | 2 | 2 | 2 |
| *P. aeruginosa* 36A | 2 | 0.5 | 4 | 1 | 2 | 2 |

The results of the table show that the compounds of the invention have a good antibacterial activitly. Particularly good result are found against *P. aeruginosa and K. Pneumoniae.* They also have good antibacterial activity against multiresistant strains. Specially good results are obtained with the compopunds of Examples 5 and 6.

**Table 5: antibacterial activity (MIC) expressed in µg/ml of comparative Examples**

| Strains | Col. | Comparative Ex. 3 | Comparative Ex. 4 | Comparative Ex. 5 | PxB |
|---|---|---|---|---|---|
| *E coli* ATCC 25922 | 0.5 | 16 | 4 | 16 | 0.25 |
| *E.coli* MB1410 | 0.5 | 2 | 4 | 8 | 0.5 |
| *K. pneumoniae* MB674 | 1 | 2 | 2 | 8 | 0.25 |
| *P. aeruginosa* ATCC 27853 | 2 | 2 | 2 | 4 | 1 |
| *P. aeruginosa* 36A | 1 | 2 | 2 | 2 | 1 |

The activities of the comparative examples have been extracted from Scientific Reports 5:10558 or from WO2017093210A1.

### Example 12: Nephrotoxicity study

Swiss CD-1 female mice weighing approximately 20-24g (JANVIER Labs) were used, housed in an individually ventilated cage system under specific pathogen-free conditions, and water and food supplied *ad libitum.* This study plan and the conduct of the study were subjected to review by the Scientific Park of Barcelona Ethical Review Process with the current study procedure Code CEEA (AR) 9377-P2. Mice were administered at a dose of 12 mg/Kg every 2 hours for a total of 6 administrations (total dose 72 mg/Kg), unless otherwise stated. After 20 h, animals were anesthesized with isoflurane, kidneys extracted and prepared for histopathological evaluation, and finally animales were sacrificed. Kidney lesions (tubule dilation, necrosis, tubule casts, cell vacuolization, cell desquamation, and cell swelling) were quantified by microscopical observation (see for instance, Roberts, ACS Infect. Dis., 2015, 1, 568). Each one of these parameters was scored with values from 0 to 4 for each animal, as a global result of observations in kidney. Partial scores have been added up to obtain a final score for each animal (Animal Score, AS). The mean of animal score has been obtained to define the Group Score (GS). The higher the score, the more nephrotoxic a compound is. The nephrotoxicity score has been determined as according to the following table of approximated values:

| Nephrotoxicity Score | Percentage of lesioned area affected |
|---|---|
| 0 | ≤5% of the section affected |
| 1 | 5-25% of the section affected |
| 2 | 25-50% of the section affected |
| 3 | 50-75% of the section affected |
| 4 | >75% of the section affected |

Tables 6 and 7 show the results of nephrotoxicity obtained for the Examples of the inventions and Table 8 for the comparative examples.

**Table 6: Summary of nephrotoxicity results in mice for the polymyxin analogues (dose of 12 mg/Kg every 2 hours x 6 administrations, subcutaneous; total dose 72 mg/Kg). The number of affected mice is indicated in the Detail box including each nephrotoxicity AS score (in parentheses). The Partial AS is the sum of the score of each mouse in each column. The Grup Score (GS) is the arithmetic mean for each AS and used as an indication of nephrotoxicity.. Group score (GS) of saline control (8 mice) was 0.**

| Compound | Number of animals | | Mice found dead | Tubular dilation | Cell vacuolization | Tubular necrosis | Tubular casts | Cell desquamation | Cloudy swelling | Animal Score (AS) | Group Score GS) |
|---|---|---|---|---|---|---|---|---|---|---|---|
| PxB | 8 | Partial AS | - | 1 | 1 | 1 | 3 | 0 | 21 | 27 | 3.4 |
| | | Detail | None | 7 mice (0) | 7 mice (0) | 7 mice (0) | 7 mice (0) | 8 mice (0) | 6 mice (3) | 4 mice (3) | |
| | | | | 1 mice (1) | 1 mice (1) | 1 mice (1) | | | 1 mice (2) | 2 mice (2) | |
| | | | | | | | 1 mice (3) | | 1 mice (1) | 1 mice (4) | |
| | | | | | | | | | | 1 mice (7) | |
| Example 5 | 8 | Partial AS | None | 0 | 0 | 0 | 0 | 0 | 3 | 3 | 0.4 |
| | | Detail | All 8 the same value (0) | | | | | | 3 mice (1) | 3 mice (1) | |
| | | | | | | | | | 5 mice (0) | 5 mice (0) | |
| | | | | | | | | | | | |
| Example 6 | 8 | Partial AS | 2 dead | 0 | 0 | 0 | 1 | 0 | 5 | 6 | 1 |
| | | Detail | 6 surviv ors | 6 mice (0) | | | 1 mice (1) | 6 mice (0) | 1 mice (2) | 1 mice (2) | |
| | | | | | | | | | 3 mice (1) | 4 mice (1) | |
| | | | | | | | 5 mice (0) | | 2 mice (0) | 1 mice (0) | |
| Example 8 | 3 | Partial AS | None | 0 | 0 | 0 | 0 | 0 | 0 | 0 | 0 |
| | | Detail | All 3 mice (0) | | | | | | | | |
| Example 9 | 3 | Partial AS | 1 dead | 0 | 0 | 0 | 0 | 0 | 0 | 0 | 0 |
| | | Detail | All 2 mice (0) | | | | | | | | |
| Example 10 | 3 | Partial AS | None | 0 | 0 | 0 | 0 | 0 | 0 | 0 | 0 |
| | | Detail | All 3 mice (0) | | | | | | | | |

**Table 7: Summary of nephrotoxicity results in mice for the polymyxin analogues dosed at 8 mg/Kg every 2 hours for a total of 6 administrations (total dose 48 mg/Kg). Group score (GS) of saline control (5 mice) was 0.**

| Compound | Number of Animals | | Mice found dead | Tubular dilation | Cell vacuolization | Tubular necrosis | Tubular casts | Cloudy swelling | AS | Group score (GS) |
|---|---|---|---|---|---|---|---|---|---|---|
| Example 5 | 5 | Partial AS | None | 0 | 0 | 0 | 0 | 0 | 0 | 0 |
| | | Detail | | - | - | - | - | - | - | |
| Example 6 | 5 | Partial AS | None | 0 | 0 | 0 | 0 | 1 | 1 | 0.2 |
| | | Detail | | - | - | - | - | 1 mouse (1) | 1 mouse (1) | |
| | | | | | | | | 4 mice (0) | | |

**Table 8: Summary of nephrotoxicity results in mice for polymyxin control and comparative examples dosed at 12 mg/Kg every 2 hours for a total of 6 sc administrations (total dose 72 mg/Kg). Group score (GS) of saline control (3 mice) was 0.**

| Compound | Number of mice | | Mice found dead | Tubular dilaion | Cell vacuolization | Tubular necrosis | Tubular casts | Cloudy swelling | AS | Group Score (GS) |
|---|---|---|---|---|---|---|---|---|---|---|
| Polymyxin B | 3 | Partial AS | none | 1 | 0 | 0 | 0 | 6 | 7 | 2.3 |
| | | Detail | | 1 mouse (1) | | | | 1 mouse (3) | 1 mouse (3) | |
| | | | | | | | | 1 mouse (1) | 1 mouse (2) | |
| | | | | | | | | 1 mouse (2) | 1 mouse (2) | |
| Comparative Example 1 | 3 | Partial AS | none | 6 | 0 | 0 | 0 | 6 | 12 | 4 |
| | | Detail | | 3 mouse (2) | | | | 1 mouse (3) | 1 mouse (5) | |
| | | | | | | | | 1 mouse (2) | 1 mouse (4) | |
| | | | | | | | | 1 mouse (1) | 1 mouse (3) | |
| Comparative Example 2 | 3 | Partial AS | 2 dead | 1 | 0 | 0 | 0 | 3 | 4 | 4 |
| | | Detail | | - | - | - | - | - | - | |
| Comparative Example 3 | 3 | Partial AS | 2 dead | 2 | | | 3 | 3 | 8 | 8 |
| | | Detail | | - | - | - | - | - | - | |
| Comparative Example 4 | 3 | Partial AS | 3 | - | - | - | - | - | - | All mice dead |
| | | Detail | dead | - | - | - | - | - | - | |
| Comparative Example 5 | 3 | Partial AS | none | 6 | 0 | 0 | 3 | 4 | 13 | 4.3 |
| | | Detail | | 1 mouse (4) | 0 | 0 | 1 mouse (3) | 2 mouse (1) | 1 mouse (8) | |
| | | | | 1 mouse (1) | | | 1 mouse (0) | 1 mouse (2) | 1 mouse (2) | |
| | | | | 1 mouse (1) | | | 1 mouse (0) | | 1 mouse (3) | |

In tables 6 and 7, it is shown that the described compounds (Examples 5-6 and 8-10) are much less nephrotoxic than polymyxin B according to the number and severity of lesions observed by histopathology of renal samples (indicated by the GS, Group Score). Polymyxin B gives a 3.4 value of GS. The compounds of the present invention give much lower values of GS, some 0 or almost 0. This means that the compounds of the invention are almost non-nephrotoxic, and some are as active as polymyxin or colistin, such as the compound of Examples 5 and 6.

In table 8, the comparative examples contain cysteine in position 10 of the sequence (not the new moiety (2*R*,3*S*)-3-amino-4-mercaptobutan-2-ol, Thiothreonine or Ttr, as the compounds of the present invention).. The comparative examples are much more nephrotoxic in the *in vivo* mice tests (nephrotoxicity GS >4) than the compounds of the invention, even more than polymyxin B.

### REFERENCES CITED IN THE APPLICATION

### Non Patent Literature

- T. Velkov et al., in "Structure- Activity Relationships of Polymyxin Antibiotics" J.Med. Chem., 2010, vol.53, pp.1898-1916;
- N. Katsuma et al., in "Development of Des-Fatty Acyl-Polymyxin B Decapeptide Analogs with Pseudomonas aeruginosa-Specific Antimicrobial Activity", Chem. Pharm. Bull., 2009, vol. 57, pp. 332-336;
- M. Vaara, Novel derivatives of polymyxins. J Antimicrob Chemother, 2013 Jun; vol. 68, pp.1213-9;
- Roberts KD, et al. Antimicrobial Activity and Toxicity of the Major Lipopeptide Components of Polymyxin B and Colistin: Last-line Antibiotics against Multidrug-Resistant Gram-negative Bacteria. ACS infect. Dis 2015, 1, 568-575;
- F. Rabanal et al.; "A bioinspired peptide scaffold with high antibiotic activity and low in vivo toxicity"; Scientific Reports 5:10558; 2015;
- Protective Groups in Organic Synthesis, Wiley-Interscience, 1999 chapter 2 and 7;
- A. I. Llobet et al., "Amino Acid- Protecting Groups", Chem Rev 2009, vol. 109, pp. 2455-2504

### Patent literature

- WO2010029196A1;
- WO2011110716A2;
- WO2014167160A1;
- WO2017093210A1.

## Claims

1. A compound of formula (I), 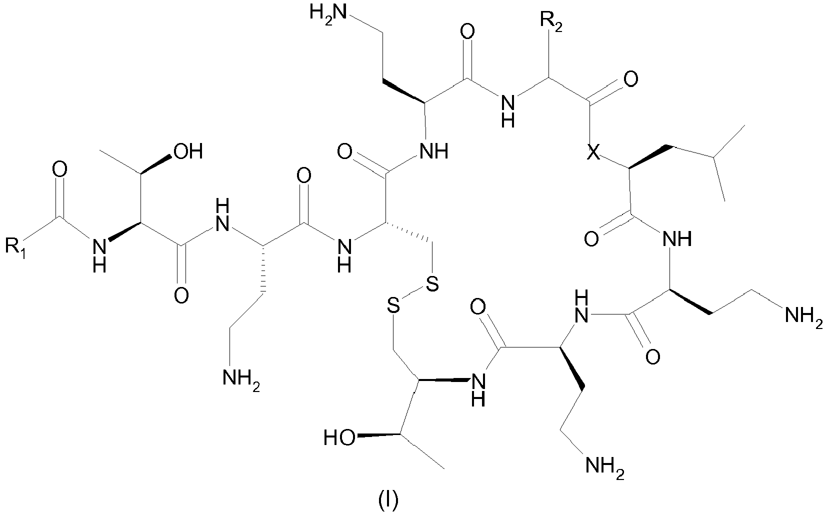 wherein: R₁ is a radical selected from the group consisting of: 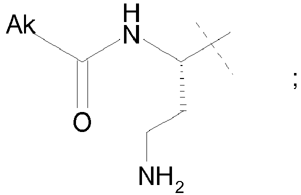 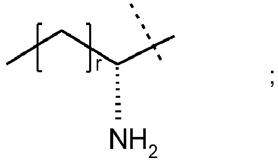 and 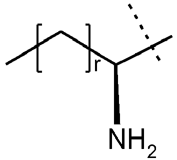
Ak is a linear or branched (C₆-C₁₁) alkyl;
r is an integer from 5 to 9;
X is NH or O; and
R₂ is selected from the group consisting of a linear or branched (C₃-C₆)-alkyl radical, phenyl, indol, 4-fluorophenyl, and pentafluorophenyl.

2. The compound according to claim 1, wherein R₁ is a radical selected from the group consisting of: 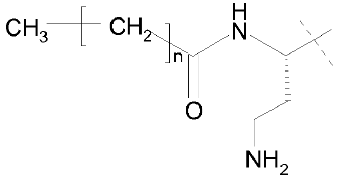 and 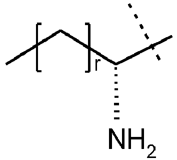 wherein n is an integer from 5 to 10, R₂ is a linear or branched (C₃-C₆)-alkyl radical or phenyl. and r and X are as defined in claim 1.

3. The compound according to claim 2, wherein R₁ is the radical below and n is 6.

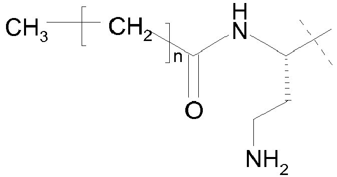

4. The compound according to claim 1, wherein R₁ is the radical below and r is 7.

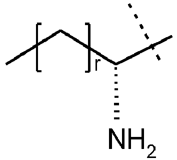

5. The compound according to any of the claims 1-4, wherein R₂ is isobutyl or phenyl.

6. The compound according to any of the claims 1-5, wherein X is NH.

7. The compound according to any of the claims 1-5, wherein X is O.

8. The compound according to claim 1, which is selected from the group consisting of:
DAdec-Thr-Dab-cyclo(S-S)[Cys-Dab-DPhe-Leu-Dab-Dab-Ttr], (Ia);
DAdec-Thr-Dab-cyclo(S-S)[Cys-Dab-DLeu-Leu-Dab-Dab-Ttr],(Ib);
Octanoyl-Dab-Thr-Dab-cyclo(S-S)[Cys-Dab-DLeu-OLe-Dab-Dab-Ttr], (Ic);
DAdec-Thr-Dab-cyclo(S-S)[Cys-Dab-DLeu-OLe-Dab-Dab-Ttr], (Id);
Octanoyl-Dab-Thr-Dab-cyclo(S-S)[Cys-Dab-DPhe-OLe-Dab-Dab-Ttr]; (Ie); and
DAdec-Thr-Dab-cyclo(S-S)[Cys-Dab-DPhe-OLe-Dab-Dab-Ttr]; (If).

9. A compound as defined in any of the claims 1-8, for use as antibacterial agent.

10. The compound according to claim 9, wherein the bacteria is selected from the group consisting of *Escherichia coli, Klebsiella pneumoniae,* and *Pseudomonas aeruginosa.*

11. A pharmaceutical composition comprising a therapeutically effective amount of a compound as defined in any of the claims 1-8, together with appropriate pharmaceutically acceptable excipients or carriers.

12. A combination of a therapeutically effective amount of a compound of formula (I) as defined in any of the claims 1-8 and a therapeutically effective amount of one or more known antibiotics, for use in the prophylaxis and/or treatment of a bacterial infection, wherein the prophilaxis and/or treatment comprises administering the antibiotics to a subject simultaneously, sequentially or separately.

13. A pharmaceutical composition comprising a therapeutically effective amount of a compound of formula (I) as defined in any of the claims 1-8 and a therapeutically effective amount of one or more known antibiotics, together with pharmaceutically acceptable excipients or carriers, which is a single dosage form; or alternatively, a kit of parts comprising a pharmaceutical composition comprising a therapeutically effective amount of the compound of formula (I) together with pharmaceutically acceptable excipients or carriers, and one or more pharmaceutical compositions comprising a therapeutically effective amount of a known antibiotic together with pharmaceutically acceptable excipients or carriers.

14. A process for preparing an intermediate compound of formula (II), wherein PG₁ is an amino protective group and PG₂ is an alcohol protective group, 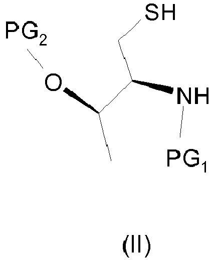 which comprises submitting a compound of formula (III) to a deprotection reaction of the thioester group reaction to yield the compound of formula (II).

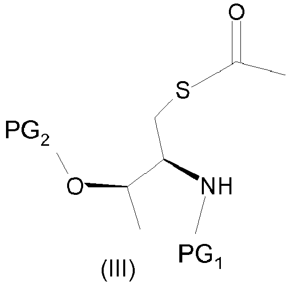

15. A compound of formula (II) wherein PG₁ is an amino protective group and PG₂ is an alcohol protective group, and wherein PG₁ and PG₂ are different protective groups.
